(19) Europäisches Patentamt
European Patent Office
Office européen des brevets

(11) Veröffentlichungsnummer: **0 176 731**
**A2**

(12) **EUROPÄISCHE PATENTANMELDUNG**

(21) Anmeldenummer: **85110358.0**

(22) Anmeldetag: **19.08.85**

(51) Int. Cl.[4]: **C 07 D 231/28**
A 61 K 31/415

(30) Priorität: **31.08.84 DE 3432062**

(43) Veröffentlichungstag der Anmeldung:
**09.04.86 Patentblatt 86/15**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Stegelmeier, Hartmut, Dr.**
**Meide 1d**
**D-4010 Hilden(DE)**

(72) Erfinder: **Fiedler, Volker, Dr.**
**Lehner Mühle 46**
**D-5090 Leverkusen 3(DE)**

(72) Erfinder: **Mardin, Mithat, Dr.**
**153 Horse Pond Road**
**Madison, CT 06443(US)**

(72) Erfinder: **Mayer, Dieter, Prof. Dr.**
**Beethovenstrasse 1**
**D-4712 Werne(DE)**

(72) Erfinder: **Perzborn, Elisabeth, Dr.**
**Am Tescher Busch 13**
**D-5600 Wuppertal 11(DE)**

(72) Erfinder: **Seuter, Friedel, Dr.**
**Moospfad 16**
**D-5600 Wuppertal 1(DE)**

(54) **Pyrazoldionderivate und ihre Anwendung bei der Bekämpfung von Krankheiten.**

(57) Die Erfindung betrifft Pyrazoldionderivate der allgemeinen Formel

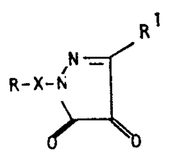

(I)

worin die Reste R, $R^1$ und X die in der Beschreibung angegebene Bedeutung haben, Verfahren zu ihrer Herstellung sowie ihre Anwendung bei der Prophylaxe und Therapie von Erkrankungen der Atemwege, Entzündungen, Rheuma, thromboembolischen Erkrankungen, Ischämien und Infarkten, Harzrhythmusstörungen und Arteriosklerose sowie Stoffgemische bzw. Arzneimittel, die solche Pyrazoldionderivate enthalten.

EP 0 176 731 A2

BAYER AKTIENGESELLSCHAFT 5090 Leverkusen, Bayerwerk
Konzernverwaltung RP
Patentabteilung Ad/ABc

# Pyrazoldionderivate und ihre Anwendung bei der Bekämpfung von Krankheiten

Die Erfindung betrifft Pyrazoldionderivate, Verfahren zu ihrer Herstellung, ihre Anwendung bei der Prophylaxe und Therapie von Erkrankungen der Atemwege, Entzündungen, Rheuma, thromboembolischen Erkrankungen, Ischämien und Infarkten, Herzrhythmusstörungen und Arteriosklerose sowie Stoffgemische bzw. Arzneimittel, die solche Pyrazoldionderivate enthalten.

Bekannte Lipoxygenasehemmer wie Nordihydroguaretsäure, 3-Amino-1-(3-trifluormethylphenyl)-pyrazolin-2, Phenidon und 5,8,11,14-Eikosatetraensäure sind entweder gleichzeitig als Cyclooxygenasehemmer oder erst bei sehr hohen Konzentrationen wirksam. Die Hemmung des Enzyms Cyclooxygenase des Arachidonsäuremetabolismus führt zu einer globalen Prostaglandinsynthesehemmung und im allgemeinen zu einer Stimulation des Lipoxygenaseweges, was Gastrotoxizität und pro-inflammatorische und asthmatische Wirkungen sowie eine erhöhte Thrombose- und Arterioskleroseneigung verursachen kann.

Außerdem haben bekannte Lipoxygenasehemmer wie 3-Amino-1-(m-trifluormethylphenyl)-pyrazolin-2 bei systemischer Verabreichung (z.B. oral) toxische Nebenwirkungen. Es besteht deshalb ein Bedarf nach stärker wirksamen Verbindungen mit einem selektiveren Wirkungsprofil ohne Nebenwirkungen.

Es wurde nun gefunden, daß bestimmte 1-substiutierte Pyrazol-4,5-dionderivate als Hemmer (Stimulatoren) von enzymatischen Reaktionen im Rahmen des Arachidonsäurestoffwechsels wirken.

Gegenstand der Erfindung sind Pyrazoldionderivate der Formel

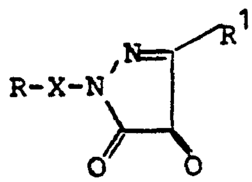

(I)

worin

R für einen Aryl- oder Heteroarylrest mit 6 bis 12 C-Atomen steht, der gegebenenfalls 1 bis 3 gleiche oder verschiedene Substituenten aus der Gruppe Halogen, Trifluormethyl, Alkyl, Alkenyl, Alkoxy, Alkylamino, Cyano, Trifluormethoxy, Nitro, Hydroxy, $SO_n$-Alkyl (n = 0 bis 2) oder $SO_n$-Trifluormethyl (n = 0 bis 2) enthält, wobei unter Alkyl, Alkenyl und Alkoxy jeweils Reste mit 1 bis 4 C-Atomen zu verstehen sind,

$R^1$ Wasserstoff oder einen Kohlenwasserstoffrest mit 1 bis 18, vorzugsweise 1 bis 8 C-Atomen, welcher gegebenenfalls eine Hydroxy-, Ether-, Ester- oder Carboxylgruppe sowie gegebenenfalls 1 bis 3 Halogenatome enthält, darstellt, und

X für eine der Gruppen

$-CH_2-CH_2-CH_2-$, $-O-CH_2-CH_2-$, $-S-CH_2-CH_2-$, $-CH_2-CH_2-$,

$-CH_2-$ oder $-\underset{\displaystyle CH_3}{\underset{|}{CH}}-$

steht, wobei das Sauerstoff- bzw. Schwefelatom an den Rest R gebunden ist.

Erfindungsgemäß bevorzugt sind Verbindungen der allgemeinen Formel (I) in der

R für einen gegebenenfalls substituierten Naphthyl-, Diphenyl- oder Phenylrest, besonders bevorzugt für einen Npahthyl- oder Diphenylrest, insbesondere für $\alpha$- oder ß-Naphthyl steht. Bevorzugt tragen diese Reste 0, 1 oder 2 Substituenten, insbesondere Br oder Cl. Bromnaphthylreste sind besonders bevorzugt.

Erfindungsgemäß werden weiterhin solche Verbindungen der allgemeinen Formel (I) bevorzugt, bei denen

$R^1$ für einen Phenyl-, Benzyl- oder Cyclohexylrest oder einen aliphatischen Kohlenwasserstoffrest, vorzugsweise einen Rest mit 1 bis 4 C-Atomen, welcher gegebenenfalls eine Hydroxyl-, Ether- oder Estergruppe aufweist, und besonders bevorzugt für eine gegebenenfalls verzweigte $C_1$-$C_4$-Alkylgruppe steht.

Erfindungsgemäß bevorzugt sind schließlich auch Verbindungen, bei welchen X für $-O-CH_2-CH_2-$, $-S-CH_2-CH_2-$, $-CH_2-CH_2-$, $-CH_2-CH_2-CH_2-$ oder $-\underset{CH_3}{\underset{|}{CH}}-$, insbesondere

für $-O-CH_2-CH_2-$, $-CH_2-CH_2-CH_2-$ oder $-\underset{CH_3}{\underset{|}{CH}}-$ steht.

Die erfindungsgemäß zur Anwendung gelangenden Pyrazoldionderivate sind zur Verhütung und Behandlung von Erkrankungen der Atemwege wie Allergien/Asthma, Bronchitis, Emphysem, Schocklunge, pulmonaler Hypertonie, Entzündungen/Rheuma und Ödemen, Thrombosen und Thromboembolien, Ischämien (periphere, kardiale, cerebrale Durchblutungsstörungen), Herz- und Hirninfarkten, Herzrhytmusstörungen, Angina pectoris, Arteriosklerose, bei Gewebetransplantationen, Dermatosen wie Psoriasis, Metastasen und zur Cytoprotektion im Gastrointestinaltrakt geeignet.

Gegenstand der Erfindung ist daher auch die Verwendung der Pyrazoldionderivate der oben aufgeführten Formel I bei der prophylaktischen und therapeutischen Behandlung des menschlichen oder tierischen Körpers.

Die erfindungsgemäß einzusetzenden Pyrazoldionderivate der allgemeinen Formel (I) können nach den folgenden Verfahren hergestellt werden.

Verbindungen der Formel (I) werden erhalten, wenn man

A) Hydrazine der Formel

$$R\text{-}X\text{-}NH\text{-}NH_2 \qquad (II)$$

in welcher

R und X die oben angegebene Bedeutung haben,

mit ß-Ketosäurederivaten der Formel

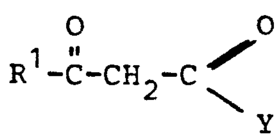

(III)

in welcher

$R^1$ die oben angegebene Bedeutung hat und

Y für einen austretenden Rest, z.B. einen Hydroxy-, Alkoxy-, Aralkoxy-, Amino- oder Alkylaminorest steht,

gegebenenfalls in Gegenwart inerter Lösungsmittel und basischer oder saurer Katalysatoren wie Alkali- und Erdalkalihydroxide und -carbonate oder wie Halogenwasserstoffsäuren, Schwefelsäure oder Sulfonsäuren, bei Temperaturen zwischen 10 und 200°C umsetzt,

oder

B) Verbindungen der Formel

R-X-A (IV)

in welcher

R und X die oben angegebene Bedeutung haben und

A einen austretenden Rest wie Halogen oder einen Dialkyloxonium-, Dialkylsulfonium- oder Trialkylammoniumrest oder einen Aryl- oder Trifluormethylsulfonsäurerest darstellt,

mit Pyrazolon-(5)-derivaten der Formel

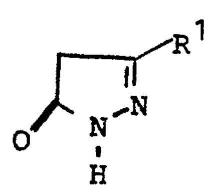

(V)

in welcher

$R^1$ die oben angegebene Bedeutung hat,

gegebenenfalls in Gegenwart inerter Lösungsmittel und anorganischer oder organischer Basen wie Alkalihydroxide, -carbonate, -alkoholate, -hydride oder -amide bei Temperaturen zwischen 10 und 200°C umsetzt,

oder

C) Acetylencarbonsäurederivate der Formel

$$Y-\overset{O}{\overset{\|}{C}}-C\equiv C-R^1 \qquad (VI)$$

in welcher

$R^1$ und Y die oben angegebene Bedeutung haben,

mit Hydrazinen der obigen Formel (II), gegebenenfalls in Gegenwart inerter Lösungsmittel und anorganischer oder organischer Basen, bei Temperaturen zwischen 50 und 200°C umsetzt, und

die nach A-C erhaltenen Pyrazolinon-Derivate mit Nitrosobenzolen der Formel VII, in dem $R^2$ und $R^3$ für einen aliphatischen Kohlenwasserstoffrest, vorzugsweise mit 1 bis 4 C-Atomen steht,

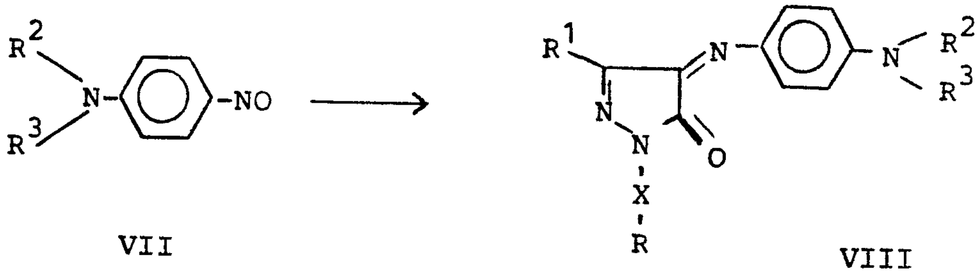

VII VIII

durch basische Katalyse in die Phenylimino-derivate der Formel VIII überführt, die mit verdünnter Säure zum Pyrazoldion verseift werden.

Die biologische Wirkung der erfindungsgemäß hergestellten Verbindungen wurde durch folgende Experimente nachgewiesen:

A) Lipoxygenasehemmung/Cyclooxygenasehemmung/Prostacyclinstimulation

1. Menschliche PMN-Leukozyten

Selektiv wird die für die Biosynthese der Leukotriene $B_4$, $C_4$ und $D_4$ verantwortlich 5-Lipoxygenase bei niedrigen Konzentrationen gehemmt.

Die polymorphkernigen Leukozyten des Menschen metabolisieren die Arachidonsäure zu 5-Hydroxy-5,8,11,14-eikosatetraensäure (5-HETE) und Leukotrien $B_4$ (5S, 12R-Dihydroxy-6 cis, 8,10-trans-14 cis-eikosatetraensäure). Die Hemmung der Freisetzung von 5-HETE und Leukotrien $B_4$ aus den Leukozyten stellt ein Maß für den lipoxygenasehemmenden Effekt der erfindungsgemäßen Verbindungen dar (vgl. Tab. 1).

Der Test mit den Humanleukozyten wurde nach Borgeat und Samuelsson (J. Biol. Chem. 254, 2643, 1979 und Proc. Natl. Adac. Sci. USA, 76, 2148, 1979) durchgeführt.

Die im vorliegenden Beispiel verwendeten Human PMN-Leukozyten ( < 95 %) wurden aus heparinisiertem Vollblut durch eine Dextran-Sedimentation und anschließende Dichtegradiententrennung (Ficoll-Paque) gewonnen (vgl. A. Boyum, Scand. J. Immunol., 5, Suppl. 5, 9, 1976).

2 x $10^7$ Zellen/ml wurden in $Ca^{2+}$-haltigem Dulbecco-Phosphat-Puffer suspendiert und in Anwesenheit bzw. Abwesenheit von Lipoxygenasehemmer mit dem Calcium-Ionophor A 23 187 stimuliert. Nach 15 Minuten wurden die Lipoxygenaseprodukte aus dem angesäuerten Inkubationsmedium extrahiert und mittels HPLC getrennt.

2. $^3$H-Arachidonsäuremetabolismus in menschlichen Thrombozyten (12-Lipoxygenaseaktivität und Cyclooxygenaseaktivität)

Der Nachweis der lipoxygenasehemmenden und cyclooxygenasehemmenden Eigenschaften der erfindungsgemäßen Verbindungen erfolgte analog der Methode von Bailey et al., Journal of Biol. Chemistry 255, 5996, 1980 und nach Blackwell und Flower, Prostaglandins 16, 417, 1978. Bei dieser Testmethode wird der Metabolismus radioaktiv markierter Arachidonsäure an gewaschenen Humanthrombozyten herangezogen. Bei diesem in vitro Test werden die radioaktiv markierten Metaboliten aus dem Reaktionsansatz extrahiert und dünnschichtchromatographisch getrennt. Das Autoradiogramm wird am Dünnschichtscanner ausgewertet. Bei diesen Testbedingungen werden die markierten Metaboliten von der nicht umgesetzten Arachidonsäure getrennt und sind anschließend quantitativ auswertbar. Die Verteilung der Radioaktivität auf die während der Metabolisierung gebildeten Cyclooxygenaseprodukte Thromboxan $A_2$ (bestimmt als $TXB_2$) und 12-Hydroxy-5,8,10-heptadekatriensäure (HHT) bzw. das Lipoxygenaseprodukt 12-Hydroxy-5,8,11,14-eikosatetraensäure (12-HETE) unter dem Einfluß der Inhibitoren stellt ein Maß für die Hemmung der Enzyme dar.

3. Prostacyclinstimulation

Außerdem stimulieren die erfindungsgemäß zu verwendenden Pyrazoldionderivate spezifisch die Synthese von Prostacyclin ($PGI_2$). $PGI_2$ wirkt im Gegensatz zu dem vasokonstriktorischen und thrombozytenaggregationsauslösenden Thromboxan gefäßdilatierend und thrombozytenaggregationshemmend.

a) Stimulation in Mikrosomen in vitro.

Die spezifische $PGI_2$-stimulierende Wirkung wurde in vitro in einem Gemisch aus Mikrosomen von Schafssamenblasen (RVSM) und Rinderaorten (BAM) gezeigt (vgl. F. Cottee et al.,

Prostaglandins, 14, 413, 1977). $^3$H-Arachidonsäure wurde in Gegenwart der Prüfsubstanzen mit einem Gemisch aus RSVM und BAM 10 Minuten bei 25°C inkubiert. Die Reaktion wurde durch Ansäuern auf pH 4,5 gestoppt. Die Fettsäuremetaboliten wurden mit Essigester extrahiert. Der Essigester wurde unter $N_2$ abgedampft und der Rückstand in $CH_3OH/CHCl_3$ (1:1) aufgenommen und auf DC-Plastikfolien aufgetragen. Die Trennung erfolgte mit einem Fließmittelgemisch Ethylenacetat/Eisessig/Isooctan/$H_2O$ (110:20:50:10; organische Phase) (P. Needleman et al., The Journal of Clinical Investigation 1978, 61, 839-849). Die Verteilung der Radioaktivität wurde mittels eines Radioscanners gemessen.

b) Stimulation in Aortenringen ex vivo.

Aortenringe von mit Prüfsubstanzen vorbehandelten Ratten werden 10 Minuten bei 37°C in Trispuffer (pH 7,8) inkubiert. Das sich bildende Prostacyclin wird nach Umwandlung zu dem stabilen 6-Keto-$PGF_{1\alpha}$ radioimmunologisch bestimmt.

Literatur:

McIntyre, D.E., Salzman, E.W., Thrombos. Haemostas. 46, 19, 1981.

Seuter, F. et al., 6th International Symposium on Atherosclerosis, Abstr. 270, Berlin 1982.

B) Leukozytenklebrigkeit

Überraschenderweise wurde auch gefunden, daß die erfindungsgemäß einzusetzenden Pyrazoldionderivate die Klebrigkeit von Leukozyten (stickiness) vermindern, d.h. deren Wanderungsgeschwindigkeit erhöhen. Die Verminderung der Leukozytenklebrigkeit trägt ebenfalls zur Abnahme ischämischer Gebiete bei, denn die Leukozyten passieren nach Behandlung mit den erfindungsgemäß einzusetzenden Verbindungen den mikrozirkulatorischen Bereich dieser Gebiete, ohne durch totalen oder zeitweiligen Verschluß von kleinen Gefäßen bzw. Kapilaren eine Minderversorgung des entsprechenden Gewebes hervorzurufen.

Die Zahl der Leukozyten, die ein Gefäß in einem bestimmten Abschnitt passieren, dient als einfaches Modell zur Messung der Klebrigkeit von Leukozyten (Bray, M.A. et al., Prostaglandins 22, 213, 1981). Die Anzahl der gezählten Leukozyten wird umso größer, je geringer die Klebrigkeit dieser Zellen wird.

Männliche Syrische Goldhamster (80-100 g) wurden mit Nembutal (i.p.; 60 mg/kg) narkotisiert. Nach Einbinden eines PE 10 Katheters in die A.femoralis wird das Tier auf eine Präparierbühne nach Duling (MVR 5, 423, 1973) gelegt und die rechte Backentasche durch Einführen eine Q-Tips herausgezogen. Sie wird vorsichtig in den dafür vorgesehenen Teil der Bühne gezogen, ausgespannt und fixiert.

Ab Beginn der Präparation wird die vorbereitete Backentasche mit 5 ml/Minute Superfusat überspült. Die Temperatur der Lösung beträgt 36°C. Unter Schonung der Gefäße wird die obere Gewebsschicht längs durchtrennt und zur Seite geklappt. Bei ca. 200facher Vergrößerung wird eine Fläche von ca. 1 $cm^2$ von Bindegewebe vorsichtig freipräpariert. Das Tier wird mit der Präparierbühne auf den Objekttisch des Mikroskops gelegt. Ein Thermofühler wird in die linke Backentasche eingeführt. Die Körpertemperatur des Tieres wird so kontrolliert und mit Hilfe einer IR-Lampe (250 Watt) auf ± 0,3°C gehalten.

Die Messung der Leukozytenklebrigkeit wird bei ca. 500facher Vergrößerung vorgenommen. In dem freipräparierten Feld wird eine Venule (30-40 µm Ø) ausgewählt. In einem bestimmten Abschnitt dieses Gefäßes wird die Anzahl der pro Zeiteinheit (Min.) vorbeiwandernden Leukozyten gezählt.

C) Koronarthrombose

Narkotisierten Hunden wurde nach linker Brusteröffnung ein Silberdraht in die linke Koronararterie eingepflanzt. Eine Elektrode wurde unter die Haut eingelegt und schloß den Stromkreis. Für 6 Stunden wurde ein Gleichstrom der geschädigten Arterie zugeführt (1). Dies führte in unbehandelten Kontrollen nach 3.2 Stunden zu einem Gefäßverschluß durch einen Plättchen-Fibrinthrombus. Man schließt das während des Experimentes aus dem Abfall des Koronarflußes und typischen Elektrokardiogrammveränderungen.

Benzothiazine wurden ca. 1 Stunde vor Beginn der Stimulierung mit Gleichstrom intraduodenal verabreicht.

Nach Versuchsende wurde das herausgeschnittene Herz mit zwei Lösungen perfundiert: Evans Blau über die Aorta zur Markierung der gesunden, unbeeinflußten Herzgebiete, und Triphenyltetrazolium Chlorid zur Bestimmung des Perfusionsgebietes der verschlossenen Arterie (2). Der Infarkt wurde in diesem Gebiet als ungefärbtes, blasses Gebiet eingeschlossen und konnte entweder durch Planimetrie der Herzscheiben oder durch Herausschneiden aus dem Herzmuskel bestimmt werden.

(1) Romson JL, Haack DW, Lucchesi BR. Elektrical induction of coronary artery thrombosis in the ambulatory canine: a mod. for in vivo evaluation of antithrombotic agents. Thromb. Res. 17, 841-853, 1980.

(2) Romson JL, Bush LR, Haack DW, Lucchesi BR. The beneficial effects of oral ibuprofen on coronary artery thrombosis and myocardial ischemia in the conscious dog. J. Pharmacol Expl. Ther 215, 271-278, 1980.

D) Herzinfarkt- und Herzrhythmusstörungen

Die Wirkungen verschiedener Pyrazoldion-derivate auf den experimentellen Herzinfarkt wurden im Hund nach akuter Unterbindung einer Koronararterie untersucht. Bei Pentobarbital-narkotisierten Hunden wurde die vordere absteigende linke Koronararterie

(LAD) proximal langsam für 6 Std. unterbunden. Die Arterie wurde nicht reperfundiert. Eine Stunde vor Ligatur wurde das Prüfpräparat in 1 %iger wäßriger Tylosesuspension intraduodenal als Bolus gegeben. In den Kontrollen wurde gleichermaßen vorgegangen, jedoch ohne Wirkstoffgabe. Die Infarktgröße und das Perfusionsgebiet der LAD wurden durch eine duale Perfusionsmethode mit Evans-Blau und Triphenyltetrazoliumchlorid dargestellt. Nach der graduellen, langsamen LAD-Unterbindung verkleinerte das 4H-1,4-Benzothiazin den Infarkt beträchtlich. Dies war zu beobachten sowohl in Bezug auf das absolute Infaktgewicht als auch in Bezug auf die Infarktgröße relativ zum linken Ventrikelgewicht. Initial sank als Folge der Koronarligatur der Blutdruck, was zur reflektorischen Herzfrequenzsteigerung führte. Sonst wurden keine durch 4H-1,4-Benzothiazin verursachten hämodynamischen Wirkungen gesehen. Die Verbindungen verminderten die infarktbedingten Erhöhungen der ST-Segmente unter der R-Zacke des peripheren EKG als Ischämiezeichen, was auf elektrophysiologische Wirkung schließen ließ.

Ergebnisse

A 1-3) Lipoxygenasehemmung in Human-PMN's; (Tabelle 1), Cyclooxygenasehemmung in Thrombozyten und Prostacyclinstimulation in Mikrosomen.

Überaschenderweise hemmen die erfindungsgemäß zu verwendenden Pyrazoldionderivate selektiv die für die Biosynthese der Leukotriene $B_4$, $C_4$ und $D_4$ verantwortliche 5-Lipoxygenase. Die 12-Lipoxygenaseaktivität wird durch die erfindungsgemäß zu verwendenden Pyrazoldionderivate bei diesen Konzentrationen nicht gehemmt.

Außerdem stimulieren die erfindungsgemäß zu verwendenden Pyrazolonderivate die Synthese von Prostacyclin ($PGI_2$).

Die Cyclooxygenase wird nicht oder erst bei sehr hohen Konzentrationen ($1x10^{-5}$ g/ml) beeinflußt.

B) Leukocytenklebrigkeit; (Tabelle 2).

Überraschenderweise wurde auch gefinden, daß die erfindungsgemäß einzusetzenden Pyrazoldionderivate die Klebrigkeit von Leukozyten (stickiness) vermindern, d.h. deren Wanderungsgeschwindigkeit erhöhen. Diese Verminderung der Leukozytenklebrigkeit trägt

ebenfalls zur Abnahme ischämischer Gebiete bei, denn die Leukozyten passieren nach Behandlung mit den erfindungsgemäß einzusetzenden Pyrazolonderivaten den mikrozirkulatorischen Bereich dieser Gebiete, ohne durch totalen oder zeitweilgen Verschluß von kleinen Gefäßen bzw. Kapillaren eine Minderversorgung des entsprechenden Gewebes hervorzurufen.

Tabelle 1: Hemmung der $LTB_4$-Synthese in menschlichen Leukozyten

| Substanz | Hemmung (%) | | | |
|---|---|---|---|---|
| | 5 µg/ml | 1 µg/ml | 0,5 µg/ml | 0,1 µg/ml |
| 6 | 100 | 88 | 60 | 38 |
| 4 | 100 | 60 | 47 | 15 |
| 8 | 100 | 35 | 10 | - |
| 10 | 100 | 56 | 12 | - |
| 2 | - | 12 | - | - |
| 7 | 100 | 21 | 10 | - |
| 3 | 100 | 37 | 20 | 6 |
| 5 | 84 | 29 | 12 | - |
| 9 | 73 | 40 | - | - |
| 11 | 32 | 29 | - | - |
| 1 | 100 | 85 | 60 | 29 |

Tabelle 2 Hemmung der Leukozytenklebrigkeit

| Substanz | Effekt nach Gabe von 1 mg/kg i.a. |
|---|---|
| 1 | Hemmung |
| 4 | Hemmung |
| 5 | keine Wirkung |
| 8 | keine Wirkung |
| 10 | Hemmung |
| 2 | Hemmung |
| 7 | Hemmung |
| 11 | Hemmung |
| 3 | kein Effekt |
| 9 | kein Effekt |
| 6 | kein Effekt |

C-D) Thrombose/Ischämie (Tab. 3)

Die erfindungsgemäß einzusetzenden Pyrazoldionderivate vermindern darüber hinaus überraschenderweise die Größe von Koronarthromben und besitzen positiven Einfluß auf Herzrhythmusstörungen. Auch diese erfindungsgemäßen Verwendungszwecke der erfindungsgemäß einzusetzenden Pyrazoldionderivate werden durch die eingangs beschriebenen, aus dem Stand der Technik bekannten Indikationen nicht nahegelegt.

Tabelle 3

| Methode | N-Hunde | Dosis | Wirkung |
|---|---|---|---|
| Reperfusions-Arrhythmie | 2 | 30 mg/kg, id | 50 % weniger als Kontrolltiere |
| Koronarthrombose | 4 | 30 mg/kg, id | 40 % kleinere Koronarthromben |

In den Arzneimitteln gemäß vorliegender Erfindung sind neben den erfindungsgemäß einzusetzenden Pyrazoldionderivaten auch pharmazeutisch unbedenkliche Verdünnungsmittel oder Trägermaterialien enthalten. Hierunter sind nicht-toxische Substanzen zu verstehen, die nach dem Vermischen mit dem Wirkstoff diesen in einer für die Verabreichung geeigneten Form liefern. Die Bezeichnung schließt vorzugsweise Wasser und üblicherweise bei der chemischen Synthese verwendete organische Lösungsmittel mit niederem Molekulargewicht aus, außer bei Vorliegen anderer pharmazeutisch erforderlicher Bestandteile wie Salze in den richtigen Mengen, um eine isotonische Zubereitung herzustellen, Puffer, oberflächenaktive Mittel, Farb- und Geschmacksstoffe und Konservierungsmittel. Beispiele für geeignete feste und flüssige Verdünnungsmittel und Trägermaterialien sind die folgenden: Wasserhaltige Puffermittel, die durch Zusatz von Glucose oder Salzen isotonisch gemacht werden können; nicht-toxische organische Lösungsmittel wie Paraffine, pflanzliche Öle, Alkohole, Glykole; gemahlene Natursteinmaterialien (beispielsweise Kaoline, Aluminiumoxide, Talkum oder Kreide); synthetische Gesteinspulver (beispielsweise hochdisperse Kieselsäure oder Silikate); Zucker; und wäßrige Suspensionen von Cellulosederivaten, z.B. Methylhydroxyethylcellulose (Tylose).

In der Regel enthalten die erfindungsgemäßen Arzneimittel 0,5 bis 95 Gew.-%, bevorzugt 1 bis 90 Gew.-%, besonders bevorzugt 5 bis 50 Gew.-%, an den erfindungsgemäß einzusetzenden Pyrazoldionderivaten.

Die orale Verabreichung kann unter Verwendung fester und flüssiger Dosierungsformen wie Pulver, Tabletten, Dragees, Kapseln, Granulat, Suspensionen, Lösungen und dergleichen vorgenommen werden. Gegebenenfalls können die Dosierungseinheiten für die orale Verabreichung mikroverkapselt werden, um die Abgabe hinauszuzögern oder über längere Zeit hinweg zu verlangsamen, wie beispielsweise durch Überziehen oder Einbetten von teilchenförmigem Material in Polymere, Wachs oder dergleichen.

Die parenterale Verabreichung kann unter Verwendung flüssiger Dosierungsformen wie steriler Lösungen und Suspensionen erfolgen, die für die subkutane, intramuskuläre oder intravenöse Injektion bestimmt sind. Diese werden durch Suspendieren oder Auflösen einer abgemessenen Menge des Wirkstoffes in einem zur Injektion geeigneten nicht-toxischen flüssigen Streckmittel wie einem wäßrigen oder öligen Medium und Sterilisierung der Suspension oder Lösung hergestellt. Stabilisatoren, Konservierungsmittel und Emulgatoren können ebenfalls zugesetzt werden.

Im allgemeinen beträgt beim Menschen die auf das Körpergewicht bezogene Tagesdosis des Wirkstoffs für die parenterale Verabreichung 0,01 bis 50 mg/kg, vorzugsweise 0,1 bis 10 mg/kg, und für die orale Verabreichung 0,1 bis 500 mg/kg, vorzugsweise 0,5 bis 100 mg/kg, besonders bevorzugt 1 bis 50 mg/kg.

Die Dosierungseinheit (Tablette, Kapsel, etc). enthält in der Regel 1 bis 100 mg, bevorzugt 5 bis 50 mg, besonders bevorzugt 10 bis 30 mg, an den erfindungsgemäß einzusetzenden Pyrazoldionderivaten.

Beispiel 1

3-Methyl-1-/2-(2-naphthyloxi)-ethyl7-2-pyrazol-4,5-dion

7,2 g (0,04 Mol) 4-Nitroso-diethylanilin wurden in 80 ml Ethanol und 10 ml Sodalösung zum Sieden erhitzt. Eine Suspension aus 10.6 g (0,04 Mol) Nafazatrom und 180 ml Ethanol und 20 ml Sodalösung wurde portionsweise zugegeben und die Mischung 14 h zum Rückfluß erhitzt. Danach engt man zur Trockne ein, nimmt den Rückstand in 600 ml $CH_2Cl_2$ auf, versetzt mit 400 ml 1/2-konz. $H_2SO_4$ und läßt über Nacht bei Raumtemperatur rühren. Nach dem Abtrennen wird die organische Phase mit Wasser gewaschen und über $Na_2SO_4$ getrocknet. Man erhält nach dem Abziehen des Lösungsmittels am Rotationsverdampfer ein rotes, öliges Rohprodukt, das mit Essigester/Ether (1:1) kristallisiert wird.

Ausbeute 2,8 g (24,8 %); Fp. 119-21°C.

Nach dem im Beispiel 1 beschriebenen Verfahren wurden folgende Substanzen der allgemeinen Formel hergestellt:

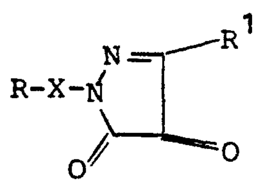

| Beisp. | R | X | $R^1$ | Fp (°C) |
|---|---|---|---|---|
| 2 | $C_6H_5$- | $-O-CH_2CH_2-$ | $-C(CH_3)_3$ | 79-81 |

| Beisp. | R | X | $R^1$ | Fp (°C) |
|---|---|---|---|---|
| 3 | 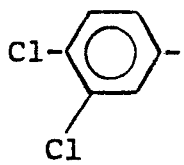 | $-CH-$<br>$CH_3$ | $-CH_2OCH_3$ | 84-85 |
| 4 | 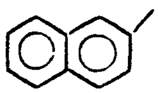 | $-O-CH_2CH_2-$ | $CH_2OCH_3$ | 96-97 |
| 5 | 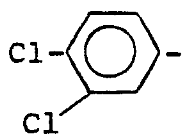 | $-CH-$<br>$CH_3$ | $-CH_3$ | 60-62 |
| 6 | 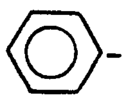 | $-OCH_2CH_2$ | $-CH_3$ | 91-92 |
| 7 | 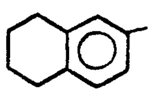 | $-CH-$<br>$CH_3$ | $-CH_3$ | amorph. |
| 8 | 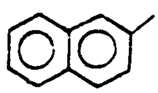 | $-O-CH_2CH_3$ | $-CH_2-$ 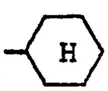 | 65-67 |
| 9 | 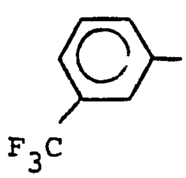 | $-CH-$<br>$CH_3$ | $-CH_3$ | amorph. |

| Beisp. | R | X | $R^1$ | Fp (°C) |
|---|---|---|---|---|
| 10 | 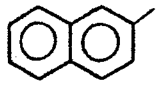 | $-O-CH_2CH_2-$ | $-CH_2CH_2CH_3$ | 79-81 |
| 11 | 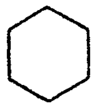 | $-\underset{CH_3}{CH}-$ | $-CH_3$ | amorph |

Patentansprüche

1. Pyrazoldionderivate der allgemeinen Formel

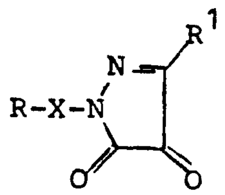

(I)

worin

R für einen Aryl- oder Heteroarylrest mit 6 bis 12 C-Atomen steht, der gegebenenfalls 1 bis 3 gleiche oder verschiedene Substituenten aus der Gruppe Halogen, Trifluormethyl, Alkyl, Alkenyl, Alkoxy, Alkylamino, Cyano, Trifluormethoxy, Nitro, Hydroxy, $SO_n$-Alkyl ($n = 0$ bis 2) oder $SO_n$-Trifluormethyl ($n = 0$ bis 2) enthält, wobei unter Alkyl, Alkenyl und Alkoxy jeweils Reste mit 1 bis 4 C-Atomen zu verstehen sind,

$R^1$ Wasserstoff oder einen Kohlenwasserstoffrest mit 1 bis 18, vorzugsweise 1 bis 8 C-Atomen, welcher gegebenenfalls eine Hydroxy-, Ether- Ester- oder Carboxylgruppe sowie gegebenenfalls 1 bis 3 Halogenatome enthält, darstellt und

X für eine der Gruppen

$-CH_2-CH_2-CH_2-$, $-O-CH_2-CH_2-$, $-S-CH_2-CH_2-$, $-CH_2-CH_2-$, $-CH_2-$ oder $-\underset{CH_3}{\underset{|}{CH}}-$

steht, wobei das Sauerstoff- bzw. Schwefelatom an den Rest R gebunden ist.

2. Pyrazoldionderivate gemäß Anspruch 1, in denen

R für einen gegebenenfalls substituierten Naphthyl-, Diphenyl- oder Phenylrest steht,

der gegebenenfalls durch 1 oder 2 Brom- oder Chloratome substituiert ist.

3. Pyrazoldionderivate gemäß Anspruch 1, in denen

$R^1$ für einen Phenyl-, Benzyl- oder Cyclohexylrest oder einen aliphatischen Kohlenwasserstoffrest steht, welcher gegebenenfalls eine Hydroxyl-, Ether- oder Estergruppe aufweist.

4. Pyrazoldionderivate gemäß Anspruch 1, in denen

X für $-O-CH_2-CH_2-$, $-S-CH_2-CH_2-$, $-CH_2-CH_2-$,

$-CH_2-CH_2-CH_2-$ oder $-CH(CH_3)-$ steht.

5. Pyrazoldionderivate gemäß Anspruch 1, in denen

R für 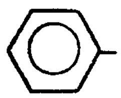 , 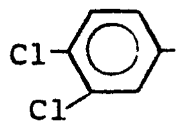 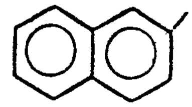

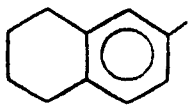 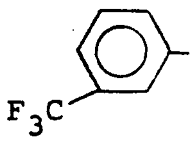 oder 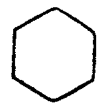 steht,

X einen Rest $-O-CH_2-CH_2-$ oder $-\underset{CH_3}{\underset{|}{CH}}-$ darstellt und

$R^1$ $-(CH_3)_3$, $-CH_2-O-CH_3$, $-CH_3$, $-CH_2-$ H ,

oder $-CH_2-CH_2-CH_3$ bedeutet.

6. Arzneimittel enthaltend Pyrazoldionderivate der Formel

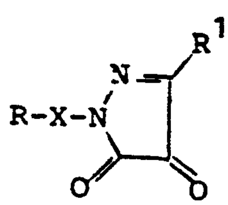 (I)

worin

R für einen Aryl- oder Heteroarylrest mit 6 bis 12 C-Atomen steht, der gegebenenfalls 1 bis 3 gleiche oder verschiedene Substituenten aus der Gruppe Halogen, Trifluormethyl, Alkyl, Alkenyl, Alkoxy, Alkylamino, Cyano, Trifluormethoxy, Nitro, Hydroxy, $SO_n$-Alkyl (n = 0 bis 2) oder $SO_n$-Trifluormethyl (n = 0 bis 2) enthält, wobei unter Alkyl, Alkenyl und Alkoxy jeweils Reste mit 1 bis 4 C-Atomen zu verstehen sind,

$R^1$ Wasserstoff oder einen Kohlenwasserstoffrest mit 1 bis 18, vorzugsweise 1 bis 8 C-Atomen, welcher gegebenenfalls eine Hydroxy-, Ether-, Ester- oder Carboxylgruppe sowie gegebenenfalls 1 bis 3 Halogenatome enthält, darstellt, und

X für eine der Gruppen

$-CH_2-CH_2-CH_2-$, $-O-CH_2-CH_2-$, $-S-CH_2-CH_2-$, $-CH_2-CH_2-$, $-CH_2-$ oder $-CH(CH_3)-$

steht, wobei das Sauerstoff- bzw. Schwefelatom an den Rest R gebunden ist.

7. Pulver, Tabletten, Dragees, Kapseln, Granulate, Suspensionen oder Lösungen enthaltend Pyrazoldionderivate der Formel

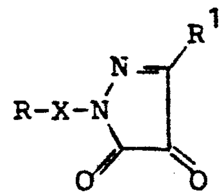

(I)

worin

R für einen Aryl- oder Heteroarylrest mit 6 bis 12 C-Atomen steht, der gegebenenfalls 1 bis 3 gleiche oder verschiedene Substituenten aus der Gruppe Halogen, Trifluormethyl, Alkyl, Alkenyl, Alkoxy, Alkylamino, Cyano, Trifluormethoxy, Nitro, Hydroxy, $SO_n$-Alkyl (n = 0 bis 2) oder $SO_n$-Trifluormethyl (n = 0 bis 2) enthält, wobei unter Alkyl, Alkenyl und Alkoxy jeweils Reste mit 1 bis 4 C-Atomen zu verstehen sind,

$R^1$ Wasserstoff oder einen Kohlenwasserstoffrest mit 1 bis 18, vorzugsweise 1 bis 8 C-Atomen, welcher gegebenenfalls eine Hydroxy-, Ether-, Ester- oder Carboxylgruppe sowie gegebenenfalls 1 bis 3 Halogenatome enthält, darstellt, und

X für eine der Gruppen

$-CH_2-CH_2-CH_2-$, $-O-CH_2-CH_2-$, $-S-CH_2-CH_2-$, $-CH_2-CH_2-$, $-CH_2-$ oder $-CH(CH_3)-$

steht, wobei das Sauerstoff- bzw. Schwefelatom an den Rest R gebunden ist.

8. Pulver, Tabletten, Dragees, Kapseln, Granulate, Suspensionen oder Lösungen enthaltend die Pyrazoldionderivate gemäß Anspruch 8 in Mengen von 0,5 bis 85 Gew.-%, bezogen auf das Trägermaterial.

9. Verwendung von Pyrazoldionderivaten der Formel

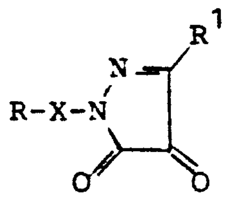

(I)

worin

R für einen Aryl- oder Heteroarylrest mit 6 bis 12 C-Atomen steht, der gegebenenfalls 1 bis 3 gleiche oder verschiedene Substituenten aus der Gruppe Halogen, Trifluormethyl, Alkyl, Alkenyl, Alkoxy, Alkylamino, Cyano, Trifluormethoxy, Nitro, Hydroxy, $SO_n$-Alkyl (n = 0 bis 2) oder $SO_n$-Trifluormethyl (n = 0 bis 2) enthält, wobei unter Alkyl, Alkenyl und Alkoxy jeweils Reste mit 1 bis 4 C-Atomen zu verstehen sind,

$R^1$ Wasserstoff oder einen Kohlenwasserstoffrest mit 1 bis 18, vorzugsweise 1 bis 8 C-Atomen, welcher gegebenenfalls eine Hydroxy-, Ether-, Ester- oder Carboxylgruppe sowie gegebenenfalls 1 bis 3 Halogenatome enthält, darstellt, und

X für eine der Gruppen

$-CH_2-CH_2-CH_2-$, $-O-CH_2-CH_2-$, $-S-CH_2-CH_2-$,

$-CH_2-CH_2-$, $-CH_2-$ oder $-CH(CH_3)-$

steht, wobei das Sauerstoff- bzw. Schwefelatom an den Rest R gebunden ist,

bei der Herstellung von Arzneimitteln.

10. Verwendung von Pyrazoldionderivaten der Formel

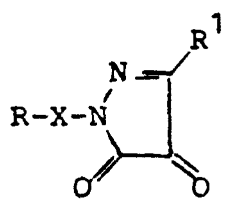

(I)

worin

R für einen Aryl- oder Heteroarylrest mit 6 bis 12 C-Atomen steht, der gegebenenfalls 1 bis 3 gleiche oder verschiedene Substituenten aus der Gruppe Halogen, Trifluormethyl, Alkyl, Alkenyl, Alkoxy, Alkylamino, Cyano, Trifluormethoxy, Nitro, Hydroxy, $SO_n$-Alkyl (n = 0 bis 2) oder $SO_n$-Trifluormethyl (n = 0 bis 2) enthält, wobei unter Alkyl, Alkenyl und Alkoxy jeweils Reste mit 1 bis 4 C-Atomen zu verstehen sind,

$R^1$ Wasserstoff oder einen Kohlenwasserstoffrest mit 1 bis 18, vorzugsweise 1 bis 8 C-Atomen, welcher gegebenenfalls eine Hydroxy-, Ether-, Ester- oder Carboxylgruppe sowie gegebenenfalls

1 bis 3 Halogenatome enthält, darstellt, und

X für eine der Gruppen

$-CH_2-CH_2-CH_2-$, $-O-CH_2-CH_2-$, $-S-CH_2-CH_2-$, $-CH_2-CH_2-$, $-CH_2-$ oder $-CH(CH_3)-$

steht, wobei das Sauerstoff- bzw. Schwefelatom an den Rest R gebunden ist,

bei der prophylaktischen oder therapeutischen Behandlung des menschlichen oder tierischen Körpers.